(19) Europäisches Patentamt
European Patent Office
Office européen des brevets

(11) Publication number: **0 563 386 A1**

(12) # EUROPEAN PATENT APPLICATION published in accordance with Art. 158(3) EPC

(21) Application number: **92901441.3**

(22) Date of filing: **19.12.91**

(86) International application number: **PCT/JP91/01734**

(87) International publication number: **WO 92/11247 (09.07.92 92/17)**

(51) Int. Cl.[5]: **C07D 251/18**, A61K 31/53

(30) Priority: **20.12.90 JP 413461/90**
**01.04.91 JP 96372/91**

(43) Date of publication of application: **06.10.93 Bulletin 93/40**

(84) Designated Contracting States: **AT BE CH DE DK ES FR GB GR IT LI LU MC NL SE**

(71) Applicant: **NIPPON SHINYAKU COMPANY, LIMITED**
**14, Kisshoin Nishinosho Monguchicho**
**Minami-ku Kyoto-shi Kyoto 601(JP)**

(72) Inventor: **MISHINA, Hitoshi 7-5, Aramaki Chuo Aoba-ku**
**Sendai-shi Miyagi 981(JP)**
Inventor: **UEDA, Fusao 272-16, Aza-choshoji Notogawacho**
**Kanzaki-gun Shiga 521-12(JP)**

(74) Representative: **Alber, Norbert et al**
**Patent- und Rechtsanwälte,**
**Hansmann Vogeser Dr. Boecker Alber Dr. Strych,**
**Albert-Rosshaupter-Strasse 65**
**D-81369 München (DE)**

(54) **ANTICANCER COMPOSITION AND COMPOUND.**

(57) An anticancer composition containing a compound represented by general formula (I) or a pharmacologically acceptable acid addition salt thereof. In formula (I), $R^{10}$ and $R^{20}$ may be the same or different from each other and each represents hydrogen, halogen, amino, aralkylamino, nitro, lower alkyl, lower alkoxy, lower alkoxyalkoxy, lower aralkyloxy or acyl; $R^{30}$ and $R^{40}$ may be the same or different from each other and each represents hydrogen, nicotinoyl, benzoyl or lower alkoxy; and n represents 0 or 1.

EP 0 563 386 A1

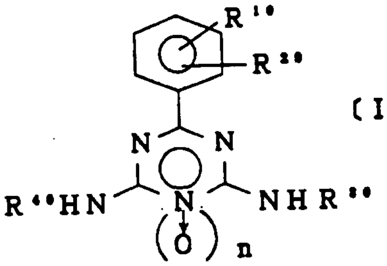

(I)

TECHNICAL FIELD

This invention relates to an anticancer composition comprising a compound of the following formula [I] or the pharmacologically acceptable acid salts thereof as an active ingredient.

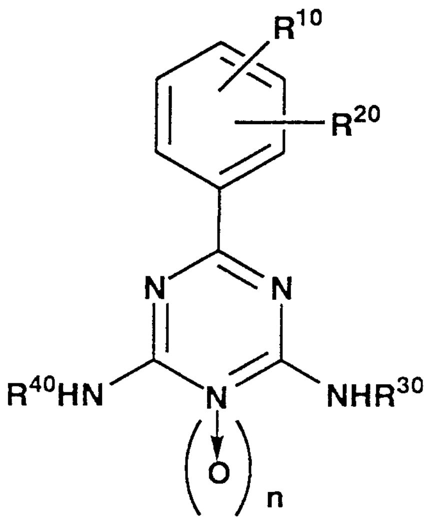

[I]

wherein $R^{10}$ and $R^{20}$ are the same or different and each represents hydrogen, halogen, amino, aralkylamino, nitro, lower alkyl, lower alkoxy, lower alkoxyalkoxy, lower aralkyloxy or acyl; $R^{30}$ and $R^{40}$ are the same or different and each represents hydrogen, nicotinoyl, benzoyl or lower alkoxy; n represents 0 or 1.

The term 'anticancer composition' is used herein to mean not only a composition of matter having its own anticancer action (an anticancer composition) but also a composition of matter which assists the anticancer action of any other drug having anticancer activity (an anticancer adjuvant composition).

Furthermore, throughout this specification, the term 'anticancer activity' is used to cover an action to prolong the survival time of a life dying of cancer.

A great majority of compounds of general formula [I], which are relevant to this invention, have peptic ulcer healing activity and antiinflammatory activity, for instance, and are already known as compounds of value as medicaments (JP Publication S59-025765 specification etc.). Some of the compounds of general formula [I] which are relevant to this invention are novel compounds not heretofore described in the literature.

BACKGROUND ART

In the clinical practice of cancer therapy today, multi-disciplinary therapeutic regimens with chemo therapy in the main are generally administered. Particularly in cases of terminal cancer, radiation therapy is indicated as an effective remedy. Radiation therapy is attracting attention as a therapeutic means for prolonging the survival time of patients with terminal cancer, the radical cure of which can hardly be expected, and suppressing the relapse and metastasis of cancer.

It is a common clinical practice to administer drugs, which are known generally as antitumor agents, as an adjunct to radiation therapy for improving the success rate of treatment, and many explorations have been made as to what antitumor agents should be drugs of choice and what should be the proper dosage (Cancer and Chemotherapy 12, 4, p.947, 1985, Journal of Medi cine and Pharmaceutical Science 13, 6, p.1755, 1985).

It is already known that, in the radiation therapy mentioned above, the concomittant use of multiple chemotherapeutic drugs in a judicious combination is rewarded by better clinical results and the inventors of the present invention had already elucidated the relationship between the types and doses of such drugs and the effect that can be achieved but any treatment modality based on this much of knowledge has not

been able to produce a dramatic effect in terminal cancer which is inherently difficult to cure.

In cancer therapies other than radiation therapy, too, the use of multiple chemotherapeutic agents in combination is a routine practice and the relationship between the types and doses of drugs chosen and the resulting effect has been studied in detail. However, it has so far been impossible to expect dramatic effects in terminal cancer for which radial cure is hard to come by.

Having been devoted to the improvement of anticancer therapy, the inventors of the present invention had been exploring for an anticancer composition which would produce dramatic effects in the resolution and healing of tumors or in the prolongation of the life span. Therefore, the object of the invention was to discover an anticancer composition capable of providing such dramatic effects.

## DISCLOSURE OF THE INVENTION

The inventors of the present invention found, though accidentally, that a compound of the general formula [I] is suited for the above-mentioned object and have arrived at the solution according to the invention.

The principle of the present invention is that a compound of general formula [I] or the pharmacologically acceptable acid salts thereof is applied as the active ingredient of an anticancer composition.

The term 'anticancer composition' is used herein to mean not only a material having anticancer activity by itself (hereinafter referred to as an anticancer composition) but also a material having an auxiliary action, for example an action to enhance the anticancer action of another drug having anticancer activity (hereinafter referred to as anticancer adjuvant composition).

In the context of the present invention, an anticancer adjuvant composition is any of (1) a substance which has no anticancer activity of its own but has an action to support the anticancer action of another drug used in combination, (2) a substance which has anticancer activity of its own and exhibits an additive effect when it is used in combination with another drug and (3) a substance which has its own anticancer activity and produces a synergistic effect when used in combination with another drug, excepting the substance which has its own anticancer activity and can be used independently as an anticancer drug.

The mechanism responsible for the claimed pharma cologic efficacy of the compound of the invention has not been elucidated as yet.

Referring, now, to general formula [I], the halogen for substituents $R^{10}$, $R^{20}$ includes chloro, fluoro, bromo and iodo.

The aralkylamino for substituents $R^{10}$, $R^{20}$ in cludes ar(e.g. phenyl)(lower)alkylamino groups, such as benzylamino, phenethylamino and phenylpropylamino.

The lower alkyl for substituents $R^{10}$, $R^{20}$ includes straight-chain and branched groups, and methyl, ethyl, propyl and butyl can be mentioned.

The lower alkoxy for substituents $R^{10}$, $R^{20}$ in cludes alkoxy groups of 1 to 6 carbon atoms, such as methoxy, ethoxy, propoxy, butoxy, pentoxy, hexyloxy, etc., and may be a straight-chain group or a branched group.

The lower alkoxyalkoxy for substituents $R^{10}$, $R^{20}$ includes those groups which are available upon substitution of a hydrogen atom of said lower alkoxy with a lower alkoxy group similar thereto. Thus, for example, methoxymethoxy, methoxyethoxy, methoxypropoxy, methoxybutoxy, ethoxymethoxy, ethoxyethoxy, ethoxypropoxy, ethoxybutoxy, ethoxypentoxy, etc. can be mentioned. The carbon chain may be linear or branched.

The lower aralkyloxy for substituents $R^{10}$, $R^{20}$ includes ar(e.g. phenyl)(lower)alkoxy groups such as benzyloxy phenylethoxy, phenylpropoxy, etc.

The acyl for substituents $R^{10}$, $R^{20}$ includes, among others, formyl, acetyl, propionyl, butyryl, valeryl, etc.

Referring, further, to general formula [I], $R^{30}$, $R^{40}$ include not only hydrogen, nicotnoyl and benzoyl but also methoxy, ethoxy, propoxy butoxy, etc. The carbon chain may be linear or branched.

The compound of general formula [I] includes compounds in which the N-atom of the triazine ring is in the oxide form, in which case n in general formula [I] represents 1.

The following is a partial list of compounds of general formula [I]. (Each figure in parentheses represents a compound number).

[100] 2,4-Diamino-6-phenyl-1,3,5-triazine
[152] 2,4-Diamino-6-(2-chlorophenyl)-1,3,5-triazine
[159] 2,4-Diamino-6-(3-chlorophenyl)-1,3,5-triazine
[153] 2,4-Diamino-6-(4-chlorophenyl)-1,3,5-triazine
[251] 2,4-Diamino-6-(2-bromophenyl)-1,3,5-triazine
[182] 2,4-Diamino-6-(3-bromophenyl)-1,3,5-triazine

[252] 2,4-Diamino-6-(2-iodophenyl)-1,3,5-triazine
[339] 2,4-Diamino-6-(2-fluorophenyl)-1,3,5-triazine
[181] 2,4-Diamino-6-(3-fluorophenyl)-1,3,5-triazine
[185] 2,4-Diamino-6-(4-fluorophenyl)-1,3,5-triazine
[257] 2,4-Diamino-6-(2-nitrophenyl)-1,3,5-triazine
Melting point 236-237 °C
[158] 2,4-Diamino-6-(3-nitrophenyl)-1,3,5-triazine
[154] 2,4-Diamino-6-(4-nitrophenyl)-1,3,5-triazine
Melting point 341-342 °C
[255] 2,4-Diamino-6-(2-methoxyphenyl)-1,3,5-triazine
[155] 2,4-Diamino-6-(4-methoxyphenyl)-1,3,5-triazine
[256] 2,4-Diamino-6-(2-ethoxyphenyl)-1,3,5-triazine
[363] 2,4-Diamino-6-(2-isopropoxyphenyl)-1,3,5-triazine
[362] 2,4-Diamino-6-(2-butoxyphenyl)-1,3,5-triazine
[353] 2,4-Diamino-6-(2-benzyloxyphenyl)-1,3,5-triazine
[345] 2,4-Diamino-6-(4-benzyloxyphenyl)-1,3,5-triazine
Melting point 225-227 °C
[378] 2,4-Diamino-6-(2-aminophenyl)-1,3,5-triazine
[352] 2,4-Diamino-6-(3-aminophenyl)-1,3,5-triazine
Melting point 212-213 °C
[347] 2,4-Diamino-6-(3-acetylphenyl)-1,3,5-triazine
Melting point 278-280 °C
[356] 2,4-Diamino-6-(2-benzylaminophenyl)-1,3,5-triazine
Melting point 162-164 °C
[167] 2,4-Diamino-6-(2,3-dichlorophenyl)-1,3,5-triazine
[160] 2,4-Diamino-6-(2,4-dichlorophenyl)-1,3,5-triazine
[169] 2,4-Diamino-6-(2,5-dichlorophenyl)-1,3,5-triazine
[170] 2,4-Diamino-6-(2,6-dichlorophenyl)-1,3,5-triazine
[156] 2,4-Diamino-6-(3,4-dichlorophenyl)-1,3,5-triazine
[166] 2,4-Diamino-6-(3,5-dichlorophenyl)-1,3,5-triazine
[400] 2,4-Diamino-6-(2,4-dibromophenyl)-1,3,5-triazine Melting point 226-227 °C
[212] 2,4-Diamino-6-(2,5-dibromophenyl)-1,3,5-triazine
[405] 2,4-Diamino-6-(3,4-dibromophenyl)-1,3,5-triazine
[220] 2,4-Diamino-6-(2,5-difluorophenyl)-1,3,5-triazine
[341] 2,4-Diamino-6-(2,6-difluorophenyl)-1,3,5-triazine
[210] 2,4-Diamino-6-(2-bromo-4-chlorophenyl)-1,3,5-triazine
[214] 2,4-Diamino-6-(2-bromo-5-chlorophenyl)-1,3,5-triazine
[213] 2,4-Diamino-6-(5-bromo-2-chlorophenyl)-1,3,5-triazine
[219] 2,4-Diamino-6-(4-bromo-3-chlorophenyl)-1,3,5-triazine
[218] 2,4-Diamino-6-(2-bromo-5-fluorophenyl)-1,3,5-triazine
[371] 2,4-Diamino-6-(2-chloro-5-iodophenyl)-1,3,5-triazine
Melting point 266-267 °C
[382] 2,4-Diamino-6-(5-chloro-2-iodophenyl)-1,3,5-triazine
[383] 2,4-Diamino-6-(5-bromo-2-iodophenyl)-1,3,5-triazine
Melting point 225-228 °C
[217] 2,4-Diamino-6-(2-chloro-5-fluorophenyl)-1,3,5-triazine
Melting point 215-216 °C
[211] 2,4-Diamino-6-(4-chloro-2-fluorophenyl)-1,3,5-triazine
[216] 2,4-Diamino-6-(5-chloro-2-fluorophenyl)-1,3,5-triazine
[215] 2,4-Diamino-6-(5-bromo-2-fluorophenyl)-1,3,5-triazine
[380] 2,4-Diamino-6-(5-chloro-2-methylphenyl)-1,3,5-triazine
[253] 2,4-Diamino-6-(2-methylphenyl)-1,3,5-triazine
[346] 2,4-Diamino-6-(3-methylphenyl)-1,3,5-triazine
[151] 2,4-Diamino-6-(4-methylphenyl)-1,3,5-triazine
[254] 2,4-Diamino-6-(2-ethylphenyl)-1,3,5-triazine
[372] 2,4-Diamino-6-(2,5-dimethylphenyl)-1,3,5-triazine
[407] 2,4-Diamino-6-(3,4-dimethylphenyl)-1,3,5-triazine
[360] 2,4-Diamino-6-(6-methyl-2-nitrophenyl)-1,3,5-triazine

[385] 2,4-Diamino-6-(2-chloro-5-methylphenyl)-1,3,5-triazine
[422] 2,4-Diamino-6-(3-chloro-4-methylphenyl)-1,3,5-triazine
[374] 2,4-Diamino-6-(2-chloro-5-nitrophenyl)-1,3,5-triazine
Melting point 297-299 °C
[368] 2,4-Diamino-6-(2-chloro-6-nitrophenyl)-1,3,5-triazine
[367] 2,4-Diamino-6-(5-chloro-2-nitrophenyl)-1,3,5-triazine
[413] 2,4-Diamino-6-(4-chloro-3-nitrophenyl)-1,3,5-triazine
[421] 2,4-Diamino-6-(3-chloro-4-methoxyphenyl)-1,3,5-triazine
[415] 2,4-Diamino-6-(4-methoxy-3-nitrophenyl)-1,3,5-triazine
[259] 2,4-Diamino-6-(3,4-dimethoxyphenyl)-1,3,5-triazine
[389] 2,4-Diamino-6-(5-chloro-2-methoxyphenyl)-1,3,5-triazine
Melting point 318-322 °C
[258] 2,4-Diamino-6-(5-bromo-2-methoxyphenyl)-1,3,5-triazine
[221] 2,4-Diamino-6-[5-chloro-2-(2-methoxyethoxy)phenyl]-1,3,5-triazine
Melting point 210-212 °C
[398] 2,4-Diamino-6-[5-bromo-2-(2-methoxyethoxy)phenyl]-1,3,5-triazine
Melting point 209-210 °C
[406] 2,4-Diamino-6-[5-chloro-2-(2-phenoxyethoxy)phenyl]-1,3,5-triazine
Melting point 202-203 °C
[338] 2,4-Diamino-6-(5-chloro-2-cyclohexyloxyphenyl)-1,3,5-triazine
[388] 2,4-Diamino-6-(2-amino-5-chlorophenyl)-1,3,5-triazine
Melting point 257-259 °C
[401] 2,4-Diamino-6-(5-amino-2-chlorophenyl)-1,3,5-triazine
[412] 2,4-Diamino-6-(3-amino-4-chlorophenyl)-1,3,5-triazine
[408] 2,4-Diamino-6-(4-amino-3-chlorophenyl)-1,3,5-triazine
[101] 2-Amino-4-nicotinoylamino-6-phenyl-1,3,5-triazine
[287] 2-Amino-4-nicotinoylamino-6-(2-chlorophenyl)-1,3,5-triazine
[289] 2-Amino-4-nicotinoylamino-6-(3-chlorophenyl)-1,3,5-triazine
[288] 2-Amino-4-nicotinoylamino-6-(4-chlorophenyl)-1,3,5-triazine
[273] 2-Amino-4-nicotinoylamino-6-(4-fluorophenyl)-1,3,5-triazine
[290] 2-Amino-4-nicotinoylamino-6-(2-iodophenyl)-1,3,5-triazine
[262] 2-Amino-4-nicotinoylamino-6-(2,5-dichlorophenyl)-1,3,5-triazine
[304] 2-Amino-4-nicotinoylamino-6-(2,6-dichlorophenyl)-1,3,5-triazine
[263] 2-Amino-4-nicotinoylamino-6-(3,4-dichlorophenyl)-1,3,5-triazine
[264] 2-Amino-4-nicotinoylamino-6-(3,4-dimethoxyphenyl)-1,3,5-triazine
[102] 2,4-Dinicotinoylamino-6-phenyl-1,3,5-triazine
[183] 2-Amino-4-ethoxyamino-6-(2,5-dichlorophenyl)-1,3,5-triazine
[103] 2-Amino-4-benzoylamino-6-phenyl-1,3,5-triazine
[425] 2-Amino-4-benzoylamino-6-(2,5-dichlorophenyl)-1,3,5-triazine
[416] 2-Amino-4-benzoylamino-6-(3,4-dichlorophenyl)-1,3,5-triazine
[104] 2,4-Dibenzoylamino-6-phenyl-1,3,5-triazine
[393] 2,4-Diamino-6-(5-bromo-2-chlorophenyl)-1,3,5-triazine N-oxide
[1001] 2,4-Diamino-6-(2,5-dichlorophenyl)-1,3,5-triazine N-oxide

The compounds of the present invention can be produced by the known methods (e.g. JP Publication No. S55-4751). As to novel compounds, examples are presented herein after for reference.

The anticancer effect and anticancer adjuvant effect of the compound of the present invention can be verified by the specific techniques detailed in the test examples which appear hereinafter, such as tumor growth inhibition assays using WiDr (human colon cancer) transplanted in an animal, Colon 26 (mouse colon cancer) and so on. In these assays, the anticancer adjuvant effect can be demonstrated by using the test compound in combination with a known anticancer drug.

In the assay of inhibitory activity against the 5-FU uptake of MDCK cells, the anticancer adjuvant activity of the test compound can be estimated based on the inhibitory effect on the uptake of a known anticancer drug.

Furthermore, in the assay of growth inhibitory activity against Colon 26, the life-prolonging effect can be evaluated from the mortality of animals during a period of 4 to 6 weeks after administration.

By the above plurality of assays, the above compounds of the present invention were confirmed to have anticancer activity and anticancer adjuvant activity.

Moreover, in clinical practice, the above com pounds of the invention were confirmed to produce tumor-remissing, tumor-healing and life-prolonging effects in patients with cancer.

The above-mentioned assays are screening systems established by NCI (National Cancer Institute) for anticancer agents. It is well-documented that anti cancer agents established to be effective by these assays are very likely to exhibit sufficient efficacy in the clinical treatment of cancer (Driscoll, J.S. Cancer Treat. Rep. 68 63-76 (1984). Goldin, A., Ven ditti J. M., DeVita, V. T. Jr. et al. Europ. J. Cancer 17 129-142, (1980). Boyd, M. R., Shoemaker, R. H., Suffness, M. et al. Seapharm Conference on "Pharma ceuticals and the Sea," Oct. pp24-25, (1985) Harber Branch Foundation, Fort Pierce, Florida.).

Among compounds of the present invention, the compounds bearing the following Compound Nos. are particularly excellent in anticancer activity and anticancer adjuvant activity.

102, 103, 104, 156, 219, 259, 262, 264, 345, 347, 352, 356, 405, 169, 159, 210, 213, 221, 263, 215, 374, 183, 255, 380, 100, 1001.

The pharmaceutically acceptable salts of compound (I) may be any of the corresponding salts of inorganic acids such as hydrochloric acid, hydrobromic acid, sulfonic acid, nitric acid, phosphoric acid, etc. and the corresponding salts of organic acids such as acetic acid, citric acid, tartaric acid, maleic acid, succinic acid, fumaric acid, p-toluenesulfonic acid, benzenesulfonic acid, methanesulfonic acid and so on.

The composition of the present invention can be used in all types of cancer therapy inclusive of chemotherapy.

When the substance of the present invention is applied as an anticancer adjuvant composition to radiation therapy, the source of radiation can be any of the sources used in the current practice of radiation therapy. Thus, γ-rays from $^{60}CO$ (cobalt), for instance, are suitable. The radiation dose may be that used heretofore in radiation therapy. By way of example, using a total dose of 25 Gy as a first target, it is appropriate to apply about 30 Gy when cisplatin is a concomitant chemotherapeutic agent or about 40 Gy in the absence of concomitant chemotherapy.

It has been found that in practicing the present invention, this radiation dose may be nearly halved to about 10-20 Gy without detracting from the predicted expect.

The chemotherapeutic agents which can be used concomitantly in the practice of the invention are known anticancer drugs and there is no particular restriction.

As such chemotherapeutic agents, there can be mentioned, in addition to the drugs used in the test examples which appear hereinafter, adriamycins such as bleomycin, doxorubicin (adriamycin), daunomycin etc., vinka alkaloids such as vincristine, vindesine, VP16, etc., 5-FU drugs such as fluorouracil etc., cytarabine, thiotepa, cyclophosphamide and so on.

It has been found that the compound of the present invention is extremely low in toxicity.

When the compound of the invention is administered as a drug, it can be used either concomitantly or in admixture with said other drug.

The compound of the invention can be administered to animals including man either as it is or in the form of a pharmaceutical composition containing 0.01 to 99.5%, preferably 0.1 to 90%, of the compound and the balance of a pharmaceutically acceptable nontoxic and inert carrier.

The carrier may be at least one member of solid, semisolid or liquid diluents, fillers and other pharma ceutical auxiliary agents. The pharmaceutical composition is preferably administered in unit dosage forms. The pharmaceutical composition of the present invention can be administered orally, into the tissue, locally (e.g. transdermally) or rectally. Of course, the dosage form suitable for each route of administration should be selected. For example, oral or parenteral (especially intravenous) administration is particularly preferred.

The dosage as an anticancer composition is preferably adjusted selected in consideration of the patient's characteristics such as age and body weight, route of administration, nature and severity of disease and so on. Usually, however, the daily dosage as the active compound for human adults is 100 μg to 30 mg/man/day, preferably 500 μg - 10 mg/man/day. Depending on cases, a lower dosage may suffice or a higher dosage may be necessary. The daily dosage may be administered in 1 to 3 divided doses.

Oral administration can be carried out using a solid or liquid unit dosage form such as a neat powder, powder, fine granule, tablet, dragee, film coated tablet, capsule, granule, suspension, solution, syrup, drop, sublingual tablet and so on.

The neat powder can be prepared by comminuting the active compound to size. The powder can be manufactured by pulverizing the active compound to size and blending the resulting powder with similar powders of one or more pharmaceutical carriers such as edible carbohydrates, e.g. starch, mannitol and so on. If necessary, flavorants or corrigents preservatives, dispersing agents, colorants, perfumes, etc. can be incorporated in the composition.

Capsules can be manufactured by preparing neat or formulated powders in the above manner or granules in the manner described hereinafter for tablets and, then, filling gelatin or other capsule shells with

the powders or granules. Prior to the filling operation, a lubricant or fluidizing agent, such as colloidal silica, talc, magnesium stearate, calcium stearate, solid polyethylene glycol, etc., can be added, each in finely divided state, to said granules. An improvement in effect of the drug administered may be obtained by adding a disintegrator or solubilizer, such as carboxymethylcellulose, carboxymethylcellulose calcium, lowsubstituted hydroxypropylcellulose, crosscarmelose sodium, carboxymethylstarch sodium, calcium carbonate, sodium carbonate and so on.

Soft-capsules can be manufactured by dispersing a powder of the compound of the invention in a vegetable oil, polyethylene glycol, glycerin or a surfactant and entrapping the dispersion in a gelatin sheet shells. Tablets can be manufactured by preparing a powdery composition with use of an excipient, molding it into granules or sags, adding a disintegrator and/or lubricant thereto and compression-molding the whole composition. The powdery composition mentioned above may be a mixture of a finely divided powder of the compound of the invention and a diluent or base such as mentioned above, optionally supplemented with a binder (e.g. sodium carboxymethylcellulose, hydroxypropyl cellulose, methylcellulose, hydroxypropylmethyl cellulose, gelatin, polyvinylpyrrolidone, polyvinyl alcohol, etc.), a dissolution retardant (e.g. paraffin, wax, hydrogenated castor oil, etc.), a reabsorption agent (e.g. quaternary ammonium salts) and an adsorbent (e.g. bentonite, kaolin, dicalcium phosphate, etc.). The powdery composition can be granulated by wetting it with a binder, such as a syrup, starch paste, gum arabic, a cellulose or polymer solution or the like, followed by stirring to mix, drying and granulating. Instead of such a granulation process, the composition may first be tableted and the resulting sags of crude form are comminuted into granules.

To the granules which are manufactured in the above manner, an appropriate lubricant, such as stearic acid, stearates, talc, mineral oil, etc., can be added for preventing the interadhesion of individual granules. The thus-lubricated composition is then compression molded.

The uncoated tablets thus obtained can be film-coated or sugar-coated.

Instead of being processed through said granulation and slagging steps, the drug may be admixed with a free-flowing inert carrier and directly compression-molded. It is also possible to utilize a transparent or translucent protective coating consisting in a hermetically sealing shellac film, and in lieu of, or on top of, said shellac film, a sugar or polymer coat and even a polished wax coat may be provided.

Other oral dosage forms such as solutions, syrups and elixers can also be provided in unit dosage forms so that the drug may be delivered in constant quantities. Syrups can be manufactured by dissolving the compound in an appropriate flavored aqueous medium, while elixers can be manufactured using a nontoxic alcoholic vehicle. Suspensions can be formulated by dispersing the compound in a nontoxic vehicle. Solu bilizers and emulsifiers (e.g. ethoxylated isostearyl alcohol, polyoxyethylene sorbitol ester), preservatives, flavorants (e.g. peppermint oil, saccharin) and other agents can also be added, where necessary.

If necessary, such a unit dosage form for oral administration may be microencapsulated. Such a formulation can be coated or embedded in a polymer, wax or the like to insure a sustained action or controlled release.

Parenteral administration can be carried out using a liquid unit dosage form, e.g. a solution or a suspen sion, which has been prepared for subcutaneous, intra muscular or intravenous administration. Such dosage form can be manufactured by suspending or dissolving a predetermined amount of the compound in an injectable nontoxic liquid vehicle such as an aqueous or oily medium and sterilizing the resulting suspension or solution. For isotonizing such an injectable pre paration, a nontoxic salt or salt solution can be added. Rectal administration can be carried out using a suppository. Such suppositors can be manufactured by mixing the compound with a low-boiling water-soluble or insoluble solid base.

## BEST MODE FOR CARRYING OUT THE INVENTION

The following working and test examples are further illustrative of the present invention.

### Example 1

2,4-Diamino-6-(4-benzyloxyphenyl)-1,3,5-triazine

a) First, 11.9 g of 4-hydroxybenzonitrile, 15.2 g of benzyl chloride, 120 ml of acetonitrile and 20.7 g of anhydrous potassium carbonate were mixed and refluxed for 5 hours. After cooling, the insoluble matter was filtered off and the solvent was distilled off under reduced pressure. The residue was recrystallized from acetonitrile and the crystals were collected by filtration and dried to provide 17.2 g of 4-benzyloxybenzonitrile as colorless crystals.

Melting point 92-94 °C.

b) To 3.7 g of the 4-benzyloxybenzonitrile thus obtained were added 2.5 g of dicyanodiamide, 37 ml of diethylene glycol dimethyl ether and 0.2 g of potassium hydroxide and the mixture was heated at 100 °C with stirring for 8 hours, After cooling, the reaction mixture was diluted with water and the resulting crystals were collected by filtration and dried. The crystals were then recrystallized from methanol, recovered by filtration and dried to provide 5.3 g of 2,4-diamino-6-(4-benzyloxyphenyl)-1,3,5-triazine as colorless crystals.

Melting point: 225-227 °C

| Elemental analysis ($C_{16}H_{15}N_5O$) | | | |
|---|---|---|---|
| | C | H | N |
| Calcd. (%) | 65.52 | 5.15 | 23.88 |
| Found (%) | 65.35 | 5.25 | 23.71 |

The following compounds could be produced in the same manner as Example 1-b).

Example 2

2,4-Diamino-6-(3-acetylphenyl)-1,3,5-triazine

Melting point: 278-280 °C

| Elemental analysis ($C_{17}H_{11}N_5O$) | | | |
|---|---|---|---|
| | C | H | N |
| Calcd. (%) | 57.63 | 4.84 | 30.55 |
| Found (%) | 57.62 | 4.81 | 30.50 |

Example 3

2,4-Diamino-6-(3-aminophenyl)-1,3,5-triazine

Melting point: 212-213 °C

| Elemental analysis ($C_9H_{10}N_6$) | | | |
|---|---|---|---|
| | C | H | N |
| Calcd. (%) | 53.46 | 4.98 | 41.56 |
| Found (%) | 53.18 | 4.91 | 41.33 |

Example 4

2,4-Diamino-6-(2-benzylaminophenyl)-1,3,5-triazine

Melting point: 162-164 °C

| Elemental analysis ($C_{16}H_{16}N_6$) | | | |
|---|---|---|---|
| | C | H | N |
| Calcd. (%) | 65.74 | 5.52 | 28.75 |
| Found (%) | 65.62 | 5.55 | 28.61 |

Example 5

2,4-Diamino-6-(2-chloro-5-nitrophenyl)-1,3,5-triazine
Melting point: 297-299 °C

| Elemental analysis ($C_9H_7ClN_6O_2$) | | | |
|---|---|---|---|
| | C | H | N |
| Calcd. (%) | 40.54 | 2.65 | 31.52 |
| Found (%) | 40.44 | 2.69 | 31.39 |

Example 6

2,4-Diamino-6-(2-amino-5-chlorophenyl)-1,3,5-triazine
Melting point: 257-259 °C

| Elemental analysis ($C_9H_9ClN_6$) | | | |
|---|---|---|---|
| | C | H | N |
| Calcd. (%) | 45.68 | 3.83 | 35.51 |
| Found (%) | 46.07 | 3.90 | 34.92 |

Example 7

Two grams of irsogladine maleate (the salt of Compound No. 169 with maleic acid) was evenly mixed with 70 g of lactose and 30 g of corn starch. After addition of 25 ml of 16% hydroxypropylcellulose solution, the mixture was granulated with stirring. After drying and size selection, 2 g of magnesium stearate and 2 g of talc were added. The mixture was then compression-molded with a rotary tablet machine.

| Formula, 110 mg per tablet | |
|---|---|
| Irosogladin maleate | 2mg |
| Lactose | 70mg |
| Corn Starch | 30mg |
| Hydroxypropylcellulose | 4mg |
| Magnesium stearate | 2mg |
| Talc | 2mg |

Example 8

To 4 mg of irsogladine maleate was added 996 mg of lactose and the mixture was evenly blended to provide a powder containing 0.4% of irsogladine maleate.

Example 9

irsogladine maleate, 100 mg, was dissolved in 80% w/w ethanol to make a total of 100 ml. This solution is used as an injection.

Example 10

Irsogladine maleate, 400 mg, was suspended in 0.5% methylcellulose-physiological saline to make a total of 100 ml. This solution is used as an injection.

Test Example 1

WiDr (human colon cancer) cells subcultured in BALB/c nude mice were dissected to prepare cubes, about 2 $mm^3$ each. These cubes were transplanted subaxillarily into separate BALB/c nude mice using a transplantation needle. The animals were divided into groups of 10 individuals on day 10 after transplantation and the body weight and the diameter of the implant were serially determined. As the compound of this invention, irsogladine maleate, 10 mg/kg, was suspended in 0.5% methylcellulose solution and administered orally once daily beginning day 14 after transplantation. Cyclophosphamide, 150 mg/kg, was dissolved in PBS and administered intraperitoneally once on day 14 after transplantation. The control group received 0.5% methylcellulose solution orally once daily beginning day 14 after transplantation.

The volume of the implant on day 18 after transplantation was divided by the volume at trans plantation. The results were shown below.

Table 1

| Test compound | Tumor volume ratio |
|---|---|
| Control | 2.00 ± 0.07 |
| Compound of the invention | 1.52 ± 0.08** |
| Cyclophosphamide | 1.52 ± 0.06** |

** $p<0.01$

The tumor volume was calculated according to the following formula, where a and b represent the minor and major axes, respectively, of the transplanted cancer.

$$\text{Tumor volume} = \frac{a^2 \times b}{2}$$

The compound of this invention at the dose of 10 mg/kg showed an effect comparable to that of cyclo phosphamide 150 mg/kg, which is a known anticancer agent. The effect of the compound of this invention as an anticancer agent is clear.

Test Example 2

(1) The homologus tumor Meth A fibrosarcoma subcultured (ascites type) in BALB/c mice, 5 x 105 cells, was transplanted subcutaneously at the flank of 6-week-old male BALB/c mice. Mice having a tumor volume of 70-100 $mm^3$ were selected and divided into groups of 9. Oral single daily administration of the test compound was started and the body weight and tumor volume were serially determined. The volume was determined using the same calculation formula as shown in Test Example 1. The compound of the invention irsogladine maleate, 10 mg/kg, suspended in 0.5% methylcellulose maleate and 5-fluorouracil, 15 mg/kg, dissolved in 0.5% methylcellulose solution were respectively administered orally. The control group received 0.5% methylcellulose solution orally. The results are shown in Fig. 1.

The compound of this invention, 10 mg/kg, showed an effect comparable to that of 5-FU, 15 mg/kg, which is a known anticancer agent. The effect of the com pound of this invention as an anticancer agent is, thus, clear.

(2) As in the above test, mice having a tumor volume of 160-180 $mm^3$ were selected and subjected to the same test as above. The compound of this invention irsogladine maleate, 10 mg/kg, 5-flurorouracil, 30 mg/kg, or as a combination therapy, irsogladine maleate, 10 mg/kg, plus 5-fluorouracil, 30 mg/kg, were administered orally. The control group received the same control material as in the above test orally. The results are shown in Fig. 2.

Both the compound of this invention and 5-FU showed a tumor-inhibitory effect but the effect was more remarkable when the two drugs were used in com bination. The anticancer adjuvant activity of the compound of this invention to 5-FU is clear.

Test Example 3

The additive effect of 5-FU derivative and the compound of this invention was tested in terms of percent tumor inhibition. Colon 26 tumor cells were transplanted subcutaneously to groups of 8 BALB/c mice and the treatment was instituted when the tumor volume amounted to about 50-100 $mm^3$. Each of the test com pound was suspended in 0.5% methylcellulose and admin istered orally in a dose of 10 mg/kg once daily for 14 days in (1) and for 21 days in (2) and (3). As 5-FU derivatives Mifurol™, Furtulon™ and Sunfural™ were used, and all the drugs were administered orally in a dose of 30mg/kg, or 90 mg/kg as total dose of three drugs. The combination group received these 5-FU derivatives concurrently with the test drug. The single drug group received each test compound alone orally. The results are shown below.

Table 2

| Test drug | | % Inhibition Single drug | % Inhibition Combination |
|---|---|---|---|
| Test (1) | 5-FU derivative, alone | 41.8 | — |
| | Compound No. 169 | 18.0 | 54.6 |
| | Compound No. 159 | 2.0 | 50.9 |
| | Compound No. 259 | 9.2 | 45.6 |
| | Compound No. 263 | 7.0 | 47.9 |
| Test (2) | 5-FU derivative, alone | 67.6 | — |
| | Compound No. 169 | 11.6 | 84.1 |
| | Compound No. 210 | — | 86.0 |
| | Compound No. 213 | — | 70.4 |
| | Compound No. 219 | 10.0 | 75.5 |
| Test (3) | 5-FU derivative, alone | 48.6 | — |
| | Compound No. 169 | 10.7 | 49.9 |
| | Compound No. 221 | 18.1 | 76.5 |
| | Compound No. 262 | 19.2 | 48.8 |
| | Compound No. 263 | 8.9 | 52.0 |

The compound of this invention not only showed an inhibitory effect when administered alone but also showed an inhibitory effect when administered in combination with 5-FU derivatives. The anticancer activity and anticancer adjuvant activity of the compound of this invention are clear.

Test Example 4

The inhibitory effect of the compound of this invention on the uptake of 5-FU in MDCK cells was investigated. MDCK cells were seeded on a 24-well culture plate. When the cells became confluent, the test compound ($10^{-6}$ - $10^{-4}$ M) and 3H-5FU (106 cpm) were simultaneously added to the medium and 1, 3 and 5 hours later, cells were harvested to determine the amount of 3H-5FU taken up with a liquid scintillation counter. The percent inhibition to control was calculated. The results were shown below.

Table 3

| Test drug | % Inhibition | | |
|---|---|---|---|
| | $10^{-4}$ M | $10^{-5}$ M | $10^{-6}$ M |
| Compound No. 102 | 62 | 59 | 86 |
| Compound No. 103 | 50 | 58 | -- |
| Compound No. 104 | 44 | 50 | 60 |
| Compound No. 356 | 51 | 73 | 91 |
| Compound No. 262 | 46 | 58 | -- |
| Compound No. 156 | 56 | 73 | 85 |
| Compound No. 219 | 60 | 69 | 86 |
| Compound No. 264 | 56 | 62 | 89 |
| Compound No. 405 | 62 | 59 | -- |
| Compound No. 352 | 64 | 69 | -- |
| Compound No. 345 | 70 | 63 | 63 |
| Compound No. 347 | 49 | 61 | -- |
| Compound No. 259 | 62 | 66 | 77 |

The anticancer adjuvant effect of the compound of this invention is clear.

Test Example 5

(1) Colon 26 tumor cells were transplanted subcutane ously in groups of six BALB/c mice and the treatment was instituted when the tumor volume amounted to about 100 - 200 $mm^3$. The test drug suspended in 0.5% methyl cellulose was administered orally in a dose of 10 mg/kg once daily during the test period. The results are shown in Fig. 3.

All animals in the control group (no test drug was contained at all) died on day 34 and all animals given 5-FU derivative died on day 40, whereas none of the animals given the compound of this invention (compound No. 169) died even after day 40.

The life-prolonging effect of the compound of this invention is clear.

(2) As in the above (1), Colon 26 tumor cells were transplanted subcutaneously in groups of six BALB/c mice and treatment was instituted when the tumor volume amounted to about 100-200 $mm^3$. The test drug suspended in 0.5% methylcellulose was administered orally in a dose of 10 mg/kg once daily during the test period. The results are shown in Fig. 4.

Eighty percent of the animals in the control group (no test drug was contained at all) died on day 63, whereas a definite life-prolonging effect was found in the animals given the compound of this invention (Compound Nos. 175, 210, 213 and 219).

Test Example 6

Compounds No. 215, 374, 183, 255, 380, 100 and 1001 manifested a strong anticancer effect in vitro test systems which the inventor used.

Test Example 7

Predetermined radiotherapy [each proven focus was irradiated with cobalt 60 gamma rays, about 28 Gy, in simply divided doses at intervals of several days and oily bleomycin was administered intramuscularly in a dose range of 0.5-3 mg/day (a total of not more than 50 mg) 30 minutes before and 30 minutes after each irradiation] was given to the following cancer patients and concomitantly the following prescription was given orally.

| | |
|---|---|
| Carmofur | 200-300 mg/day x 2 |
| Doxifluridine | 200-100 mg/day x 2 |
| Tegafur | 200 mg/day x 2 |
| Irsogladine maleate | 2 mg/day x 2 |

The results are shown below.

(1) Cases of maxillary cancer and epipharyngeal cancer

(1) A 61-year-old man with left maxillary cancer.
(2) A 75-year-old woman with epipharyngeal cancer.
(3) A 55-year-old woman with cancer of external anditory cancer. (4) A 52-year-old woman with right external auditory cancer. (5) A 59-year-old woman with cancer of hard palate. (6) A 67-year-old man with maxillary cancer. (7) A 61-year-old woman with maxillary cancer. (8)** A 49-year-old man with epipharyngeal cancer. (9) A 58-year-old woman with epipharyngeal cancer.

In the above nine cases, the disappearance of tumor was confirmed by palpation or diagnostic imaging 4 weeks after initiation of treatment. In the patient indicated by a double asterisk, the disappearance of tumor was confirmed histologically by biopsy.

(2) Cases of rectal cancer

(1) A 74-year-old man. (2) A 62-year-old man. (3) A 58-year-old man. (4)** A 54-year-old man. (5)** A 75-year-old man.

In the above five cases, the disappearance of tumor was confirmed by palpation or diagnostic imaging 4 weeks after initiation of treatment. In the patients each indicated by a double asterisk, the disappearance of tumor was confirmed histologically by biopsy.

(3) Cases of pharyngeal cancer and cancer of upper esophagus

(1) A 60-year-old man with hypopharyngeal cancer.
(2) A 58-year-old man with cancer of upper esophagus.
(3)** A 59-year-old man with cancer of upper esophagus.
(4)** A 69-year-old man with tongue cancer.
(5) A74-year-old man with cancer of upper esophagus.
(6) A61-year-old man with hypopharyngeal cancer.
(7) A65-year-old man with pharyngeal cancer.
(8)** A52-year-old man with pharyngeal cancer.
(9) A 69-year-old man with pharyngeal cancer.
(10) A 52-year-old man with pharyngeal cancer with lung metastasis.
(11) A 67-year-old man with cancer of middle esophagus.
(12) A 84-year-old man with middle esophagus.
(13) A 84-year-old man with floor-of-mouth cancer.

In the above 13 cases, the disappearance of tumor was confirmed by palpation or diagnostic imaging 4 weeks after initiation of treatment. In the patients each indicated by a double asterisk, the disappearance of tumor was confirmed histologically by biopsy.

(4) Cases of breast cancer

(1) A 67-year-old woman. (2)** A 65-year-old woman. (3)** A 68-year-old woman. (4)** A 58-year-old woman. (5)** A 44-year-old woman. (6) A 61-year-old woman. (7)** A 73-year-old woman. (8)** A 58-year-old woman with right breast dancer. (9)** A 54-year-old woman with left breast cancer. (10) A 60-year-old woman. (11) A 48-year-old woman with right breast cancer.

In the above 11 cases, the disappearance of tumor was confirmed by palpation or diagnostic imaging 4 weeks after initiation of treatment. In the patients each indicated by a double asterisk, the disappearance of tumor was confirmed histologically by biopsy.

(5) Cases of bladder cancer, renal cancer, ovarian cancer, etc.

(1) A 70-year-old man. (2) A 80-year-old man. (3) A 77-year-old man. (4) A 42-year-old man. (5) A 77-year-old man with prostatic cancer. (6) A man with ureteral cancer.

In the above six cases, the disappearance of tumor was confirmed by palpation or diagnostic imaging 4 weeks after initiation of treatment.

(6) Case of stomach cancer

(1) A 77-year old man. The disappearance of tumor was confirmed by diagnostic imaging 4 weeks after initiation of treatment.

In all of the 45 cases, the disappearance of tumor was confirmed by palpation or diagnostic imaging, and in 14 cases of them, the disappearance of tumor was histologically verified by biopsy after confirmation by palpation or the like.

In radiotherapy in combination with any of the above-mentioned chemotherapeutic agents excepting irsogladine maleate, tumor-eliminating effect such as the above was never found. Thus, the effect of the compound of this invention is clear.

Test Example 8

Following Test Example 7, the clinical effect was investigated. Radiotherapy, chemotherapy and administration of irsogladine maleate were performed in the same manner as in Test Example 7. The results are as follows.

(1) Laryngeal cancer/hypopharyngeal cancer

(1) A 57-year-old man experienced pharyngalgia in 1989 and hoarseness in June 1990. In December of the same year, SCC of the pharynx metastasized to the right neck was confirmed. Chemotherapy was given simultaneously with irradiation. In February 1991, the administration of irsogladine maleate for about 15 days resulted in complete resoltuion of the tumor. The patient survives.

(2) A 76-year-old man complained of hoarseness at the beginning of 1988 and pharyngeal obstruction at the beginning of 1990. In December 1990, SCC of the pharynx metastasized to the left neck. Administration of irsogladine maleate in combination with radiation therapy and chemotherapy resulted in complete resolution of the tumor. The patient survives.

(3) A 61-year-old man had stiffness of the right shoulder at the beginning of 1990 and hoarseness and venous dilation of the right upper arm in August, and SCC of the right neck due to lymph node metastasis from pharyngeal cancer with lung metastasis in December 1990. Administration of irsogladine maleate as an adjunct to radiotherapy and chemotherapy resulted in complete resolution of the tumor. The patient survives.

(4) A 50-year-old man had hoarseness at the biginning of 1989 and dyspnea due to SCC of the anterior association of the vocal cord and due to pharyngeal cancer. Administration of irsogladine maleate in combination with radiotherapy and chemotherapy resulted in complete resolution of the tumor. The patient survives.

(5) A 64-year-old man underwent polypectomy for the first time around 1967 and thereafter 3 years apart on three occasions. In October 1990, swelling of the right arm and metastasis to the mediastinum occurred. The diagnosis of SCC of the pharynx was made. Administration of irsogladine maleate in combination with radiotherapy and chemotherapy resulted in complete resolution of the tumor. The patient survives.

(6) A 79-year-old man having hoarseness of three years' duration. Bloody sputum occurred in July 1989. In September 1990, a tumor was found in the anterior association of the vocal cord and the diagnosis of SSC was made. Chemotherapy was given in combination with radiation therapy. The subsequent administration of irsogladine maleate in combination with radiotherapy and chemotheray resulted in partial resolution of the tumor. The patient survives.

(7) A 58-year-old man developed leukocytosis and hoarseness in April 1990. In July 1990, polyps of the vocal cord were found. The diagnosis of SCC of the pharynx was made. Administration of irsogladine maleate in combination with radiotherapy and chemo therapy resulted in complete resolution of the tumor. The patient survives.

(2) Urinary cancer

(1) A 59-year-old man. In the summer of 1989, right low back pain and hematuria developed. The diagnosis of TCC of the ureter was made in October 1990. After resection, the administration of irsoglandine maleate in combination with radiotherapy and chemotherapy was started in January 1991, which resulted in complete resolution of the tumor. The patient survives.

(2) A 71-year-old man. In November 1989, TCC of the ureter of infiltrating type. Chemotherapy was given concomitantly with irradiation. About 4 months after resection, tumor relapsed on the right low back. Chemotherapy was given concomitantly with irradiation. Thereafter, the administration of irsogladine maleate in combination with radiotherapy and chemotherapy was started in September 1990, which resulted in partial resolution of the tumor. The patient survives.

(3) Tongue cancer, floor-of-mouth cancer, and cancer of hard palate

(1) A 52-year-old man. In August 1990, SCC of the left margin of the tongue. After resection, metastasis to the left neck. Thereafter, the administration or irsogladine maleate in combination with radiotherapy and chemotherapy resulted in complete resolution of the tumor. The patient survives.

(2) A 29-year-old woman. After insertion of artificial teeth in 1988, the left margin of the tongue was elevated. At the beginning of 1990, the patient was diagnosed as having SCC of the tongue with metastasis to the left neck. In December 1990, difficulty in eating was complained of. Administration of irsogladine maleate in combination with radiotherapy and chemotherapy resulted in complete resolution of the tumor. The patient survives.

(4) Recurrent breast cancer

(1) A 57-year-old woman. In the summer of 1978, right breast cancer, adrenocarcinoma, was found. In December 1978, radiotherapy and chemotherapy were instituted and resection was performed. In 1983, relapse. Resection. Sternal metastasis. Radiotherapy and chemotherapy were instituted. In 1988, metastases to the left breast, axilla and bone were found and chemotherapy as well as radiotherapy was instituted and rsection performed. In the autumn of 1990, bone metastasis occurred. Administration of irsogladine maleate in combination with radiotherapy and chemotherapy was started. In 1990, the patient died of injury of cervical vertebra and paraplegia.

(2) A 54-year-old woman. In November 1989, right breast cancer was resected. Invasive duct carcinoma. From the spring of 1990, aphagia persisted. In October 1990, administration irsogladine maleate in combination with radiotherapy and chemotherapy was started, which resulted in complete resolution of the tumor. The patient survives.

(5) Breast cancer (first on-set)

(1) A 54-year-old woman. In November 1990, breast cancer was noticed. Immediately, radiotherapy and chemotherapy were instituted and at the same time, irsogladine maleate was administered for 3 months. In December 1990, metastasis was confirmed. In January 1991, resection was performed. In February of the same year, complete resolution of the tumor was noted. The patient survives.

(6) Maxillary cancer and epipharyngeal cancer

(1) A 17-year-old man. In December 1986, the diagnosis of SCC of the epipharynx was made and irradiation and chemotherapy were instituted and continued. In October 1990, metastases to the lung and

lumbar vertera occurred. Administration of irsogladine maleate in combination with the radiotherapy and chemotherapy resulted in partial resolution of the tumor. The patient survives.

(2) A 72-year-old woman. In 1990, the patient was diagnosed as having SCC of epipharynx with metastasis to the right neck. The administration of irsogladine maleate in combination with radiotherapy and chemothrapy resulted in complete resolution of the tumor.

(3) A 61-year-old man. Since the beginning of 1989, the patient had undergone radiation therapy and three resections. In August 1990, pulmonary metastasis from epipharynx SCC was found. Irsogladine maleate was administered concomitantly with radiotherapy and chemotherapy. By September 1990, the tumor had been reduced in size and become cavitated. The tumor disappeared in October 1990. In December 1990, mixed infection occurred. In January 1991, the patient died.

(4) A 50-year-old man. In the summer of 1990, diplopia and easy fatigability developed. In October 1990, antiinflammatory therapy was instituted. In December 1990, irsogladine maleate was administered in combination with irradiation and chemotherapy. In January 1991, visual acuity improved. In February 1991, the tumor was partially resolved. The patient survives.

(7) Cancer of the external auditory canal

(1) A 55-year-old man. In 1988, the patient underwent repeated resection for the tumor of the right anterior ear and SCC of parotid gland. At the fourth resection, a metastasis to the right external auricle was found. In January 1991, the tumor of the right anterior ear was irradiated. In combination with the radiation therapy, chemotherapeutic agents and irsogladine maleate were administered. The tumor disappeared in February 1991. The patient survives.

(2) A 51-year-old woman. Diagnosed as having SCC of the right external auditory canal. In September 1990, treatment with irsogladine maleate was started concomitantly with radiotherapy and chemotherapy. In November 1990, resection was performed. In February 1911, complete resolution occurred. The patient survives.

(3) A 61-year-old man. In 1979, otorrhea occurred. Later, exploratory excision was performed. In November 1989, cancer of the left middle ear was found. Chemotherapy in combination with radiotherapy was given. Resection. In November 1990, SCC relapsed in the left anterior ear. Irsogladine maleate was administered in combination with radiotherapy and chemotherapy. In January 1991, resection was performed. The cholesterol mass completely disappeared. The patient was living as of February 1991.

(8) Metastatic lung cancer

(1) A 75-year-old man. The primary lesion was rectal cancer. ADC. In December 1990, a shadow in the right lower lung field was suspected. Administration of irsogladine maleate was instituted in combination with radiotherapy and chemotherapy. By January 1991, the dotted shadow of the right lower lung field had been reduced to 1/4. The patient survives.

(2) A 60-year-old man. The primary lesion was hypopharynx cancer. SCC. In July 1989, resection was performed. Chemotherapy as well as radiotherapy was given. The shadow was scattered in both lung fields and pleural effusion was noted. In November 1990, treatment with irsogladine maleate was started concomitantly with radiotherapy and chemotherapy. In January 1991, the tumor disappeared completely. The patient survives.

(3) A 74-year-old man. The primary lesion was esophageal cancer (upper). SCC. In January 1989, irsogladine maleate was administered concomitantly with radiotherapy and chemotherapy. Erythromycin was effective. The tumor disappeared completely. The patient survives.

(4) A 61-year-old man. The primary lesion was pharyngeal cancer. SCC. In 1989, right cervical lymph node enlargement was noted. Hoarseness. In December 1990, metastases to hili of the lung. Treatment with irsogladine maleate in combination with radiotherapy and chemotherapy was instituted. In January 1991, the enlargement of the pharynx and of the hili of the lung disappeared. The patient survives.

(5) A 47-year-old man. The primary lesion was pancreatic carcinoma. Carcinoid. In 1988, low back pain occurred. In 1990, pulmonary metastasis. Treatment with irsogladine maleate in combination with radiotherapy and chemotherapy was given. In December of the same year, reduction of the metastatic focus was noted. In 1991, the tumor disappeared completely. The patient survives.

(6) A 54-year-old man. The primary lesion was colon cancer. ADC. In September 1987, resection was performed. In February 1990, bone metastasis occurred in the right waist. Pulmonary metastasis. Treatment with irsogladine maleate in combination with radiotherapy and chemotherapy was instituted. In 1991, the metastatic lung cancer was reduced in size. Partial resolution. The patient survives.

(9) Terminal cancer

(1) A 42-year-old woman. In 1988, resection revealed ovarian cancer. ADC. In June 1990, recurrence was found. Administration of irsogladine maleate in combination with chemotherapeutic agents was instituted. The tumor remained unchanged. The patient was alive as of February 1991.

(2) A 77-year-old woman. In 1987, an abdominal tumor was noticed and resection was performed. In August, the tumor proved to be ovarian cancer. ADC. In November 1990, metastases to the right and left Virchow's nodes were found. Treatment with irsogladine maleate was instituted as an adjunct to radiotherapy and chemotherapy. In December 1990, an abdominal tumor was noticed at the cicatrized resection site and, therefore, radiotherapy, 2 Gy/day was given for 3 weeks while administration of irsogladine maleate and chemotherapeutic agent was continued. In 1991, the tumor disappeared completely. The patient survives.

(3) A 77-year-old woman. Uterine cancer. SCC. Uremia supervened. After renal catheterization, irsogladine maleate was administered in combination with radiation therapy (40 Gy) and chemotherapy. In February 1991, partial resolution of the tumor was obtained. The patient survives.

(4) A 40-year-old woman. In October 1989, stomach cancer with ascites, which was tubular ADC, was found. At the beginning of 1990 after resection, treatment with irsogladine maleate was given in combination with irradiation and chemotherapy. In November 1990, the patient died of DIC.

(10) Colorectal cancer

(1) A 59-year-old man. Since 1988, the patient had had constipation. In April 1990, exploratory excision was performed. RK. Tubular ADC. Treatment with irsogladine maleate in combination with radiotherapy and chemotherapy was instituted. Bladder compression, perforation of the intestine, and intestinal obstruction. In September 1990, the patient died of mixed infection.

(2) A 51-year-old man. In 1988, melena. In November 1990, RK. ADC. Pulmonary metastasis. Virshow's nodal metastasis. Ascites. Artificial anus. Resection. In February 1991, treatment with irsogladine maleate was instituted in combination with radiotherapy and chemotherapy. The ascites were reduced and the patient became ambulant. The patient survives.

(3) A 56-year-old man. In August 1990, tumor of the right hypogastrium, CK and ADC. In January 1991, irsogladine maleate was administered in combination with radiotherapy and chemotherapy. In February 1991, ascites were removed. The patient survives.

(11) Sarcoma

(1) A 70-year-old woman. Liposarcoma of the left chest wall with metastasis to the right mediastinum. Mixoid type. In September 1990, a tumor of the left chest wall was noticed. In November 1990, resection was performed. In December of the same year, resection was performed again. In January 1991, treatment with irsogladine maleate was given in combination with radiotherapy and chemotherapy. Phlebectasia of the right upper arm. In February 1991, the phlebectasia of the right upper arm improved. Deformity of the mediastinum was relieved. Partial resolution. The patient survives.

(2) A 68-year-old man with pelvic chondrosarcoma. In 1978, excision of tumor (first excision) was performed. In 1983, a second excision. In 1987, a third excision. In November 1990, treatment with irsogladine maleate was given in combination with radiotherapy and chemotherapy. The patient was living as of February 1991.

(3) A 58-year-old man with chondrosarcoma of the right leg. After resection, administration of chemotherapeutic agents was repeated together with irradiation. In February 1991, pulmonary metastasis was detected. Treatment with irsogladine maleate was given in combination with radiotherapy and chemotherapy. Pain was relieved.

(4) A 65-year-old man. In the spring of 1990, a tumor was detected in the left subauricular region. In August 1990, with the diagnosis of malignant lymphoma differs large cell, chop was performed but the tumor increased in size. Irradiation was not effective. In December 1990, treatment with irsogladine maleate was given in combination with radiotherapy and chemotherapy. The tumor was reduced from 4 x 4 cm to one-half in 11 days and to 2 x 2 cm in 13 days. In February 1991, no scar was observed. Complete resolution. The patient survives.

(5) A 65-year-old man. Resection was repeated several times. Malignant multiple schwannoma vertebrae. In October 1990, a tissue section was taken on resection. MR disclosed a focus. Treatment with irsogladine maleate was given in combination with radiotherapy and chemotherapy. In February 1991,

paralysis of limbs was found. The patient survives.

Of the above 39 patients, only four died and there were no patients in whom complete or partial resolution was not obtained. It is evident that the compound of this invention had a dramatic tumor-eliminating effect.

BRIEF DESCRIPTION OF THE DRAWINGS

Fig. 1 shows the results of Test Example 2-(1). The open circle stands for control, the open triangle for 5-FU and the closed circle for the compound of the invention. The tumor volume ($mm^3$) is plotted on the vertical axis and the number of days after drug administration on the horizontal axis.

Fig. 2 shows the results of Test Example 2-(2). The open circle stands for control, the open triangle for F-5U, the closed circle for the compound of the invention, the open square for the combination therapy. The tumor volume ($mm^3$) is plotted on the vertical axis and the number of days after drug administration on the horizontal axis.

Fig. 3 shows the results of Test Example 5. The open circle stands for control, the open square for the F-5U derivative group and the closed square for the group given the compound of this invention. The survival rate (%) is plotted on the vertical axis and the number of days after drug administration on the horizontal axis.

Fig. 4 shows the results of Test Example 5-(2). The open circle stands for the control group, the open square for Compound No. 175 group, the closed circle for Compound No. 210 group, the open triangle for Compound No. 213 group and the closed square for Compound 219 group. The survival rate (%) is plotted on the vertical axis and the number of days after drug administration on the horizontal axis.

**Claims**

**1.** An anticancer composition comprising a compound of the following formula [I] or the pharmacologically acceptable acid salts thereof as an active ingredient.

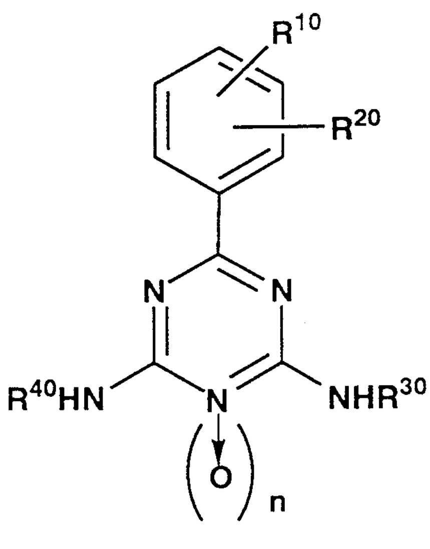

[I]

wherein $R^{10}$ and $R^{20}$ are the same or different and each represents hydrogen, halogen, amino, aralkylamino, nitro, lower alkyl, lower alkoxy, lower alkoxyalkoxy, lower aralkyloxy or acyl; $R^{30}$ and $R^{40}$ are the same or different and each represents hydrogen, nicotinoyl, benzoyl or lower alkoxy n represents 0 or 1.

**2.** An anticancer composition comprising a compound of the following formula [I] or the pharmacologically acceptable acid salts thereof as an active ingredient.

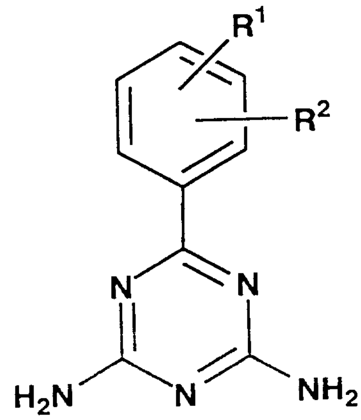

[II]

wherein R1, R2 are the same or different and each represents hydrogen or halogen.

**3.** Any compound selected from the group consisting of a compound of the formula

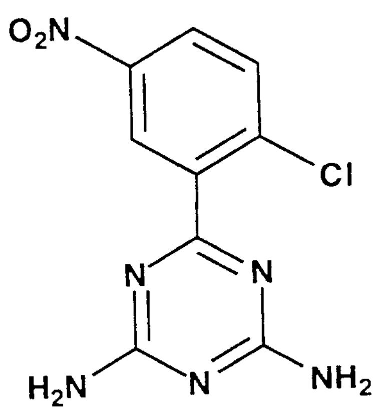

,

a compound of the formula

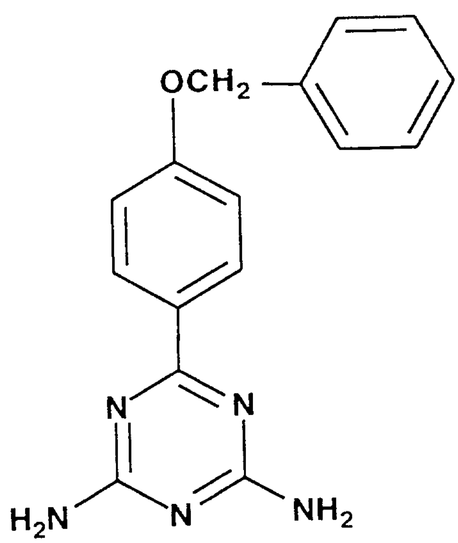

a compound of the formula

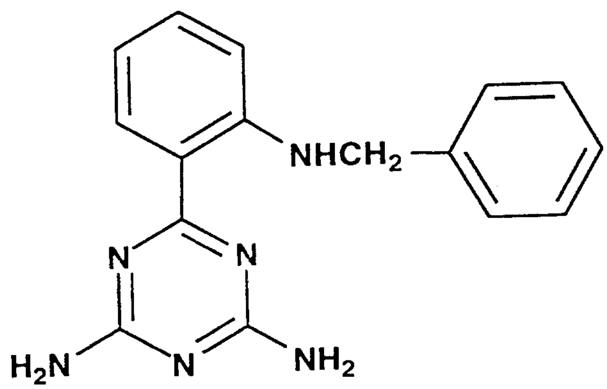

,

a compound of the formula

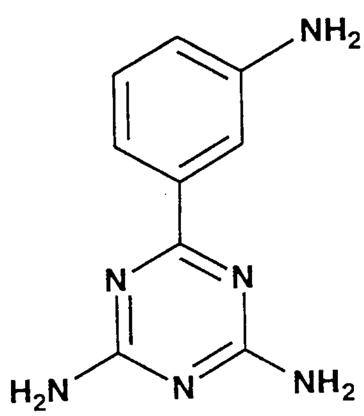

,

a compound of the formula

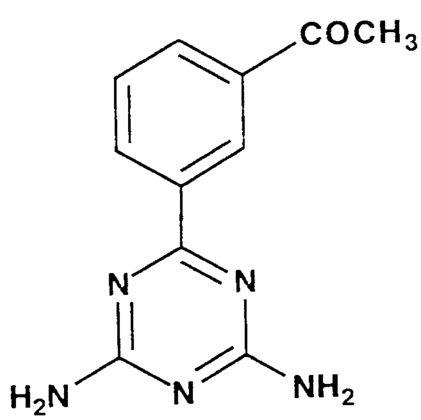

,

and a compound of the formula

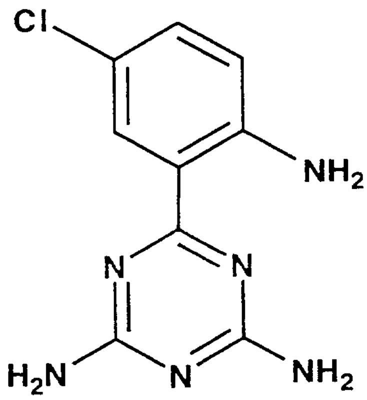

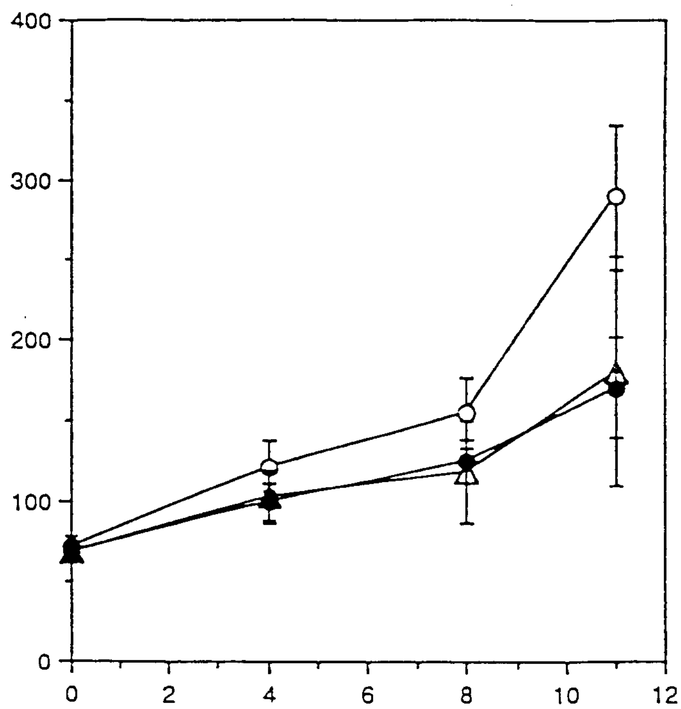


Fig. 1

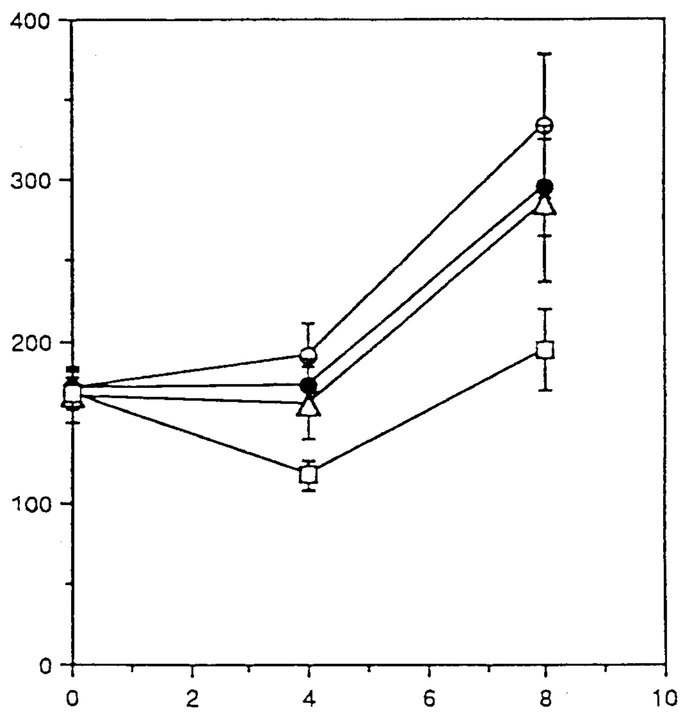


Fig. 2

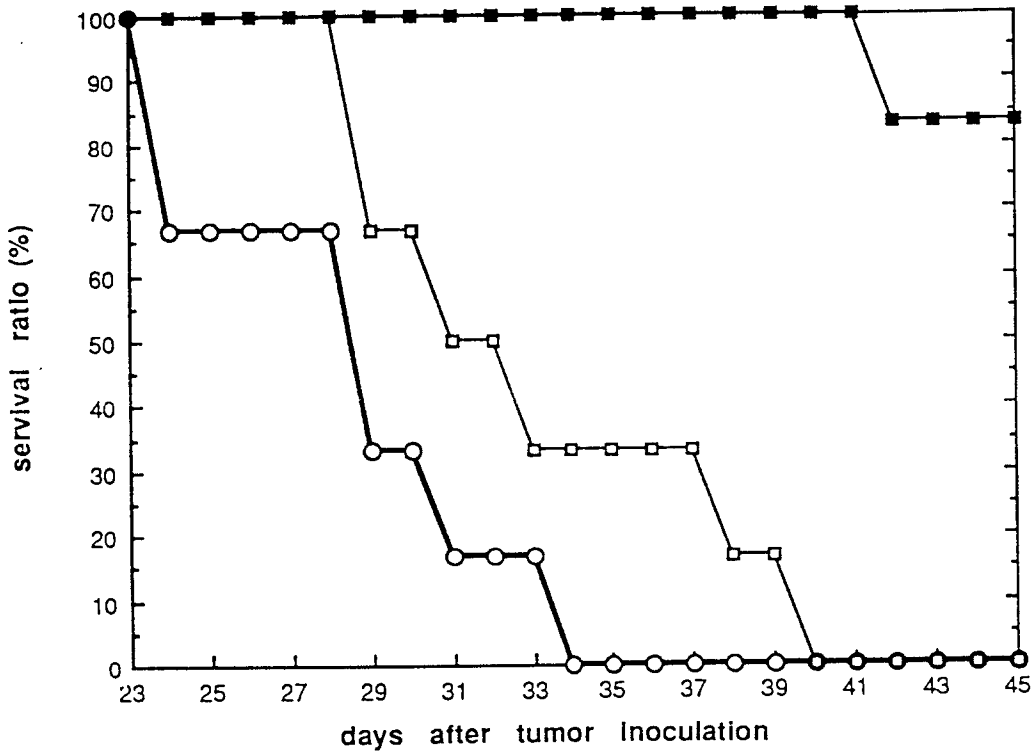
100
90
80
70
60
50
40
30
20
10
0
servival ratio (%)
23
25
27
29
31
33
35
37
39
41
43
45
days after tumor inoculation

Fig. 3

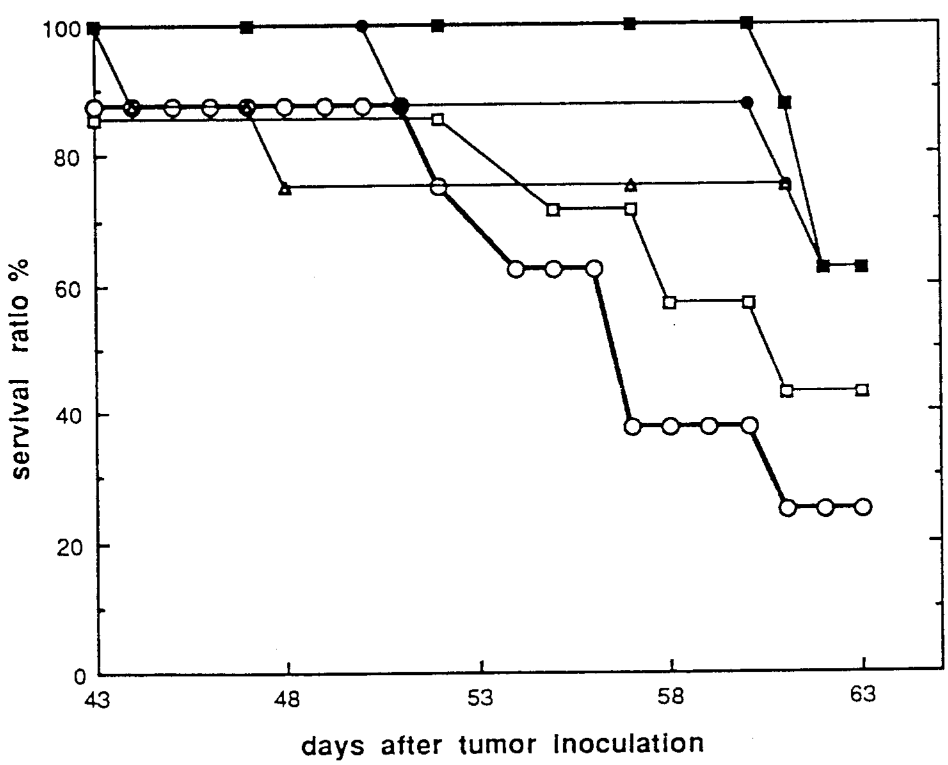
100
80
60
40
20
0
servival ratio %
43
48
53
58
63
days after tumor inoculation

Fig. 4

# INTERNATIONAL SEARCH REPORT

International Application No PCT/JP91/01734

**I. CLASSIFICATION OF SUBJECT MATTER** (if several classification symbols apply, indicate all) [6]

According to International Patent Classification (IPC) or to both National Classification and IPC

Int. $Cl^5$ C07D251/18, A61K31/53

**II. FIELDS SEARCHED**

Minimum Documentation Searched [7]

| Classification System | Classification Symbols |
|---|---|
| IPC | C07D251/18, A61K31/53 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US, A, 4,532,238 (Nippon Shinyaku Co., Ltd.), July 30, 1985 (30. 07. 85), Lines 19 to 47, column 1 | 1-3 |
| A | US, A, 4,873,242 (Nippon Shinyaku Co., Ltd.), October 10, 1989 (10. 10. 89), ABSTRACT | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 16, 1992 (16. 03. 92) | April 7, 1992 (07. 04. 92) |
| **International Searching Authority** | **Signature of Authorized Officer** |
| Japanese Patent Office | |